# EUROPÄISCHE PATENTSCHRIFT

(11) **EP 2 526 992 B1**
(45) Veröffentlichungstag und Bekanntmachung des Hinweises auf die Patenterteilung: **29.07.2020**
(21) Anmeldenummer: 12168242.1
(22) Anmeldetag: 16.05.2012
(51) Int. Cl.: A61M 25/00, A61F 5/44, A61M 39/28

(54) **Katheterventil**
Catheter valve
Soupape de cathéter

(30) Priorität: 27.05.2011 CH 9122011
(43) Veröffentlichungstag der Anmeldung: 28.11.2012
(73) Patentinhaber: Profiform AG, 6280 Hochdorf (CH)
(72) Erfinder: Müller, Kurt, 6294 Ermensee (CH)
(74) Vertreter: Schneider Feldmann AG Patent- und Markenanwälte

(56) Entgegenhaltungen:
- EP-A1- 0 150 666
- WO-A1-93/24173
- WO-A1-2010/048649
- US-A- 6 162 201

## Beschreibung

Die vorliegende Erfindung ein Katheterventil, insbesondere ein selbstschliessendes Katheterventil zur Verwendung in der Urologie, Geriatrie u. a.

Katheterventile sind in vielfacher Ausführungsform bekannt und erhältlich. Aufgrund der zumeist langen Verwendungszeiten müssen sie ergonomisch geformt und leicht sein, dazu einfach und möglichst einhändig bedienbar sein. Eine weitere Anforderung besteht in einem möglichst niedrigen Preis.

Ein solches Katheterventil ist aus der EP-B-0597058 bzw. der WO 93/24173 A1 bekannt.

Es ist ein selbstschliessendes Katheterventil, das ein Gehäuse mit ovalem Querschnitt und mit einem konischen Schlauchaufsteckstutzen umfasst, auf den ein Katheterschlauch aufschiebbar ist, und umfasst weiterhin ein Ventilschlauchstück, das das Gehäuse zumindest teilweise durchsetzt. Vorgesehen ist ein Betätigungsorgan, mit dem das Ventil in seine Offenstellung bringbar ist und das auf ein Federelement wirkt. Das Betätigungsorgan ist weitgehend innerhalb von dessen Aussenkontur im Gehäuse angeordnet und ist translatorisch in Richtung der Längsachse des Querschnittovals dergestalt, dass bei Bedienung des Betätigungsorgans der Ventilschlauch seine Offenstellung einnimmt.

Das Gehäuse umschliesst eine Kammer, in der das Federelement durch Verformung des Ventilschlauchstücks dieses verschliesst. Das Federelement ist eine V-förmig gebogene Feder, deren einer Schenkel vollflächig ist und an der Innenwandung der Kammer in ganzer Länge aufliegt, während der andere und bewegliche Schenkel eine Ausnehmung in der Breite des Ventilschlauchstücks aufweist, die von einem endverstärkten Steg begrenzt ist. Mit dem Steg ist der Ventilschlauch an der gegenüberliegenden Wand der Kammer quetschbar, wobei das an der Schmalseite des Gehäuses angeordnete Betätigungsorgan ein Knopf ist, der auf zwei, den endständigen Steg tragenden Längsstegen der Ausnehmung auf den beweglichen Schenkel wirkt.

Infolge des offenbarten Quetschens des Ventilschlauchs kann einerseits ein Überdrücken des Federelements, ohne Rückfederung in Ausgangslage und andererseits ein klemmendes Eindrücken oder gar Beschädigen des Ventilschlauchs in der Ausnehmung resultieren. Dies insbesondere dann, wenn der Ventilschlauch durch Ablagerungen in der fliessenden Flüssigkeit, z. B. Urin eine geringere Flexibilität aufweist. Die US 6162201 zeigt einen Urinkatheder mit einem Sperrventil, wobei das Ventil durch einen Drucktaster betätigt wird, der keine horizontale Verschieblichkeit zulässt.

Dies ergibt ein hohes Risiko bezüglich verkanten/klemmen.

Der Schlauch kann nicht durch den Katheder geführt werden und muss an Stutzen mit dem Ventil verbunden werden, was als Sicherheitsmangel anzusehen ist.

Mittels einer Feder, die einen Taster aufnimmt, wird auf ein Element gedrückt, welches den Schlauch sperrt.

*Aus der* EP-A-0150666 *ist ein Verschluss an einem Harnkatheder* ersichtlich, der ein durch Fingerdruck betätigbares Auslassventil mit einem elastisch verformbaren Schlauch aufweist. Der Schlauch ist durch ein Gehäuse des Auslassventils geführt und wird ebenfalls durch Federkraft zwischen einer taste und einer Klemme gedrückt.

Bei einem medizinischen Instrument, insbesondere ein Pleurapunktionskatheder mit selbstschliessendem Ventil gemäss WO-A-2010048649 ist ein verschiebbarer Verschlusskörper in einer Kammer vorgesehen, durch den eine Hohlnadel geführt ist. Der Verschlusskörper ist in Schliessrichtung von einem Spannelement vorgespannt und wird in Offenstellung der Hohlnadel von dieser gehalten.

Ein weiteres Katheterventil, welches über eine Anschlusspartie mit einer Kupplungsvorrichtung verbindbar ist, wird in der DE-A-19718582 vorgeschlagen. Die Kupplungsvorrichtung soll ein wieder lösbares Verbinden einer weiteren Schlauchverbindung an das Ventil ermöglichen.

Der Erfindung liegt daher die Aufgabe zugrunde, ein Katheterventil, insbesondere ein selbstschliessendes Katheterventil zur Verwendung in der Urologie, Geriatrie u. a. zu entwickeln, das die Nachteile des Standes der Technik meidet und eine höhere Funktionssicherheit ermöglicht. Die Aufgabe ist mit den kennzeichnenden Merkmalen des Patentanspruchs 1 gelöst. Seitliche Schultern am Federelement in Form einer Kippfeder zur Führung des Betätigungsorgans verhindern dessen vollständiges Abdrücken und somit eine dauerhafte Verformung der Kippfeder, die einen Schlauchverschluss verunmöglichen würde.

Vorteilhafte Ausführungsformen sind in den abhängigen Ansprüchen offenbart. So sind erfindungsgemäss beide Schenkel der Kippfeder vollflächig ausgeführt und ermöglichen eine klemmungsfreie Anlage des Ventilschlauchs.

Schenkel des Betätigungsorgans sind ausreichend lang und erreichen den Grund der Kammer, bevor das Federelement vollständig abgedrückt werden kann. Und sie sind in den seitlichen Schultern der Kippfeder geführt.

Weiterhin ist das Betätigungsorgan bevorzugt ein Druck-Schiebetaster. Ein Deckel des Gehäuses ist vorzugsweise kraft- und/oder formschlüssig und lösbar mit diesem verbunden, wodurch sich die Sanitation und Wiederverwendbarkeit verbessert. Ein Mittel zur zusätzlich translatorischen Einwirkung auf den Ventilschlauch bewirkt die Ablösung eventueller Ablagerungen und damit eine Verlängerung der Einsatzzeit ohne Demontage und Reinigung. Weitere Möglichkeiten der Schlauchdichtung und des Schlauchanschlusses werden ebenfalls vorgeschlagen.

Die Erfindung wird nachfolgend in einem Ausführungsbeispiel anhand einer Zeichnung näher beschrieben. In der Zeichnung zeigen die:

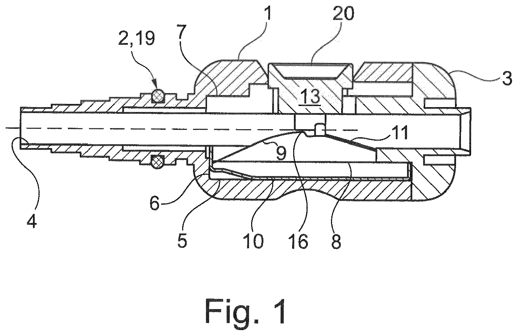

- Fig. 1: einen Längsschnitt durch das Katheterventil

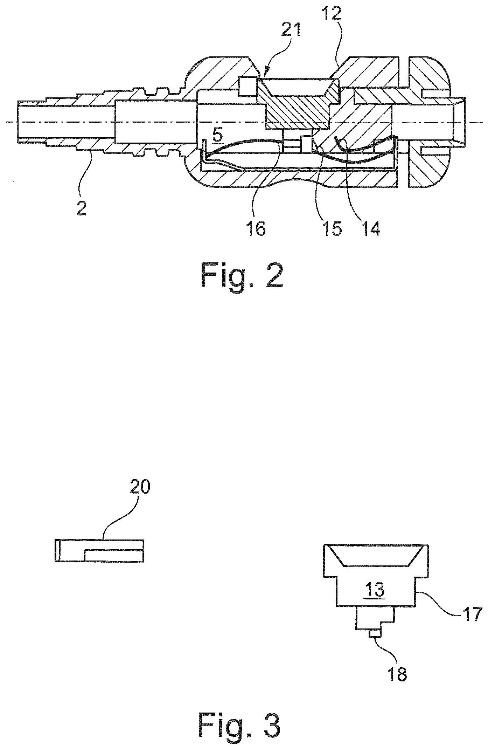

- Fig. 2: das Katheterventil mit gedrückter Kippfeder
- Fig. 3: das Betätigungsorgan im Schnitt

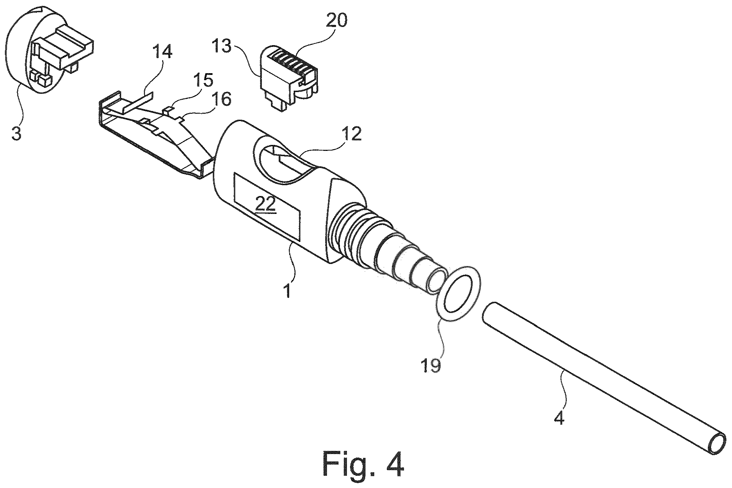

- Fig. 4: eine vereinfachte Montagedarstellung.

Die für das erfindungsgemässe Katheterventil wesentliche äussere Formgebung ergibt sich bereits aus der EP-B-0597058. Die äussere Gestalt des Katheterventils wird wesentlich durch ein zylindrisch-ovales Gehäuse 1 mit einer konischen, zylindrischen Spitze, einem Schlauchaufsteckstutzen 2 bestimmt. Auf den Schlauchaufsteckstutzen 2 kann ein Kathederschlauch abgedichtet aufgeschoben werden. Gehäuse 1 und Schlauchaufsteckstutzen 2 sind bevorzugt einstückig mittels Spritzgiessens hergestellt.

Am gegenüberliegenden Ende des Gehäuses 1 ist ein Deckel 3 montiert, der wie der Schlauchaufsteckstutzen 2 eine zylindrische Öffnung für die fluchtende Durchführung eines Ventilschlauchs 4 aus Silikon o. dgl. aufweist, wie dies bereits aus der EP-B-0597058 hervorgeht. Der Deckel 3 ist mittels Klebverbindung oder kraft-/formschlüssig mit dem Gehäuse 1 verbindbar.

Der Deckel 3 bildet zugleich die Abschlusswand einer, von der äusseren Gestaltung unabhängigen Kammer 5, die einen im wesentlichen rechteckigen Querschnitt mit angrenzenden Nuten 6, 7 besitzt. In der Nut 6 ist eine Kippfeder 9 in Form einer V-förmig gebogenen Feder mit vollflächigen Schenkeln 10, 11 angeordnet, dessen fester Schenkel 10 auf dem Grund der Nut 6 liegt.

An der Wand der gegenüberliegenden Schmalseite des Gehäuses 1 ist eine Öffnung 12 für ein Betätigungsorgan 13 vorgesehen. Wie bereits in der EP-B-0597058 offenbart ist, wird durch diese Gestaltung eine unbeabsichtigte Betätigung des Ventils vermieden und durch farbliche Hervorhebung des Betätigungsorgans 13 wird eine korrekte Bedienung des Ventils erleichtert.

Die Kippfeder 9 resp. dessen Schenkel 10, 11 sind etwas breiter als der Durchmesser des Ventilschlauchs 4, der zumindest teilweise auf dem oberen, flexiblen Schenkel 11 aufliegt. Der Schenkel 11 ist vorgespannt zwischen gebogenen Endstücken des Schenkels 10 angeordnet. Der Schenkel 10 bildet dadurch ein weites U und fungiert somit auch als Halterung für den Schenkel 11. An einem Endstück ist ein kurzer Schliessfederarm 14 mit einem Quersteg angelenkt, der im belasteten Zustand, d. h. beim Drücken des Betätigungsorgans 13 mitschwenkt, so dass der Ventilschlauch 4 vollständig freigegeben wird. Im unbelasteten Zustand hingegen drückt der Steg des Schliessfederarms 14 den Ventilschlauch 4 vollständig gegen die Kammerwand über dem Federelement 9.

Neben dieser Schliessfunktion bewirkt die Kippfeder 9 ein Verschieben des Betätigungsorgans 13 in seine Ausgangslage zurück, so dass der Ventilschlauch 4 nur während des Drückens des Betätigungsorgans 13 offen resp. durchlassfähig ist. Um bei Bedarf, z. B. nachts, eine ständige Öffnung des Ventils zu erreichen, kann das Betätigungsorgan 13 mit einem Verriegelungsschieber o. dgl. versehen sein, der das Betätigungsorgan 13 in der gedrückten Position hält. Hierbei wird der Schieber 20 in gedrückter Position des Betätigungsorgans 13 teilweise in eine Ausnehmung 21 im Gehäuse 1 verschoben.

Die Kippfeder 9 ist bevorzugt aus Federbandstahl gefertigt. Der Schenkel 11 der Kippfeder 9 weist auf beiden Längsseiten zwei, voneinander beabstandete Schultern 15, 16 auf, die unterschiedlich geformt sein können.

Das Betätigungsorgan 13 weist seitliche Schenkel 17 mit verlängerten Stirnflächen 18 auf. Die Schenkel 17 sind im Zwischenraum zwischen den Schultern 15, 16 der Kippfeder 9 geführt um ein Verkanten des Betätigungsorgans 13 beim Betätigen zu vermeiden. Beim Betätigen werden die Schenkel 17 in Richtung der Nut 6 der Kammer 5 bewegt, bis die Stirnflächen 18 auf seitlichen Stegen aufsitzen. Dieser Weg ist kürzer als der maximale Federweg des flexiblen Schenkels 11 der Kippfeder 9, so dass dieses immer zurückfedern kann. Bei Führung entsprechender Flüssigkeiten kann weiterhin ein Mittel 22, z. B. seitlich am Gehäuse 1 angebracht, zur zusätzlich translatorischen Einwirkung auf den Ventilschlauch die Ablösung eventueller Ablagerungen bewirken, z. B. als Entkalkungsschieber und damit eine Verlängerung der Einsatzzeit ohne Demontage und Reinigung ermöglichen.

### Bezugszeichenliste:

- 1: Gehäuse
- 2: Schlauchaufsteckstutzen
- 3: Deckel
- 4: Ventilschlauch
- 5: Kammer
- 6: Nut
- 7: Nut
- 8: Stegfläche
- 9: Kippfeder
- 10: Schenkel
- 11: Schenkel
- 12: Öffnung
- 13: Betätigungsorgan
- 14: Schliessfederarm
- 15: Schulter
- 16: Schulter
- 17: Schenkel
- 18: Stirnfläche
- 19: Dichtring
- 20: Schieber
- 21: Ausnehmung
- 22: Mittel

## Patentansprüche

1. Katheterventil, insbesondere ein selbstschliessendes Katheterventil zur Verwendung in der Urologie, Geriatrie u. a., umfassend ein Gehäuse (1) mit ovalem Querschnitt und mit einem konischen Schlauchaufsteckstutzen (2), auf den ein Katheterschlauch aufschiebbar ist, weiterhin umfassend ein Ventilschlauchstück, das das Gehäuse (1) zumindest teilweise durchsetzt, ein Betätigungsorgan (13), mit dem das Ventil in seine Offenstellung bringbar ist und das auf ein Federelement wirkt und weitgehend innerhalb der Aussenkontur im Gehäuse (1) angeordnet ist und in Richtung der Längsachse des Querschnittovals dergestalt bedienbar ist, dass bei Bedienung des Betätigungsorgans (13) der Ventilschlauch seine Offenstellung einnimmt, und dass das Gehäuse (1) eine Kammer (5) umschliesst, in der das Federelement durch Verformung des Ventilschlauchstücks dieses verschliesst, wobei das Federelement eine gebogene Feder, eine Kippfeder (9) ist, deren einer Schenkel an der Innenwandung der Kammer (6) in ganzer Länge aufliegt, **dadurch gekennzeichnet, dass** die Schenkel (10, 11) des Federelements vollflächig sind und dass der flexible Schenkel (11) auf beiden Längsseiten Schultern (15, 16) aufweist.

2. Katheterventil nach Anspruch 1, **dadurch gekennzeichnet, dass** auf beiden Längsseiten des Schenkels (11) je zwei, voneinander beabstandete Schultern (15, 16) vorgesehen sind.

3. Katheterventil nach Anspruch 1 oder 2, **dadurch gekennzeichnet, dass** das Betätigungsorgan (13) seitliche Schenkel (17) mit verlängerten Stirnflächen (18) aufweist, die im Zwischenraum zwischen den Schultern (15, 16) des Schenkels (11) des Federelements geführt sind.

4. Katheterventil nach Anspruch 1 oder 2, **dadurch gekennzeichnet, dass** ein Deckel (3) vorgesehen ist, der kraft- und/oder formschlüssig mit dem Gehäuse (1) verbindbar ist.

5. Katheterventil nach Anspruch 4, **dadurch gekennzeichnet, dass** der Schlauchaufsteckstutzen (2) mit wenigstens einem Dichtring (19) versehen ist.

6. Katheterventil nach Anspruch 1 oder 2, **dadurch gekennzeichnet, dass** ein Mittel (22) seitlich am Gehäuse (1) angebracht ist und in Längsrichtung auf den Ventilschlauch (4) einwirkt.

7. Katheterventil nach mindestens einem der Ansprüche 1 bis 3, **dadurch gekennzeichnet, dass** das Federelement eine Kippfeder (9) ist.

## Claims

1. A catheter valve, in particular a self-closing catheter valve for use in urology, geriatrics and other disciplines, comprising a housing (1) having an oval cross-section and a conical tube attachment stub (2) onto which a catheter tube can be pushed, further comprising a valve tube member which at least partially passes through the housing (1), an actuating element (13), with which the valve can be brought into its open position and which acts on a spring element and is arranged largely inside the outer contour in the housing (1) and is operable in the direction of the longitudinal axis of the oval cross-section to such effect that then the actuating element (13) is operated the valve tube assumes its open position, and that the housing (1) encloses a chamber (5) in which the spring element closes off the valve tube member by deformation thereof, wherein the spring element is a curved spring, a tilt spring (9) of which the entire length of one leg is braced on the inner wall of the chamber (6),
**characterized in that** the legs (10, 11) of the spring element are flush, and that the flexible leg (11) has shoulders (15, 16) on both longitudinal sides thereof.

2. The catheter valve according to Claim 1, **characterized in that** two shoulders (15, 16) each are provided on both longitudinal sides of the leg (11) and are positioned at a distance from one another.

3. The catheter valve according to Claim 1 or 2, **characterized in that** the actuating element (13) has lateral legs (17) with elongated front faces (18), which are guided in the intermediate space between the shoulders (15, 16) on the leg (11) of the spring element.

4. The catheter valve according to Claim 1 or 2, **characterized in that** a cover (3) is provided, which is connectable to the housing (1) by force-locking and/or form-locking means.

5. The catheter valve according to Claim 4, **characterized in that** the tube attachment stub (2) is furnished with at least one sealing ring (19).

6. The catheter valve according to Claim 1 or 2, **characterized in that** a means (22) is attached to the side of the housing (1) and acts on the valve tube (4) longitudinally.

7. The catheter valve according to at least one of Claims 1 to 3, **characterized in that** the spring element is a tilt spring (9).

## Revendications

1. Soupape de cathéter, en particulier soupape de cathéter à auto-fermeture destinée à être utilisée en urologie, gériatrie, entre autres, comprenant un logement (1) à section ovale et comprenant une tubulure conique d'insertion de tube (2) sur laquelle un tube de cathéter peut être poussé, comprenant en outre une pièce de tube de soupape qui traverse au moins partiellement le logement (1), un organe d'actionnement (13) avec lequel la soupape peut être amenée dans sa position ouverte et qui agit sur un élément de ressort et est disposé dans le logement (1) dans une large mesure à l'intérieur du contour extérieur et peut être utilisé en direction de l'axe longitudinal de l'ovale de section de telle façon que lors de l'utilisation de l'organe d'actionnement (13), le tube de soupape adopte sa position ouverte, et que le logement (1) entoure une chambre (5) dans laquelle l'élément de ressort, par déformation de la pièce de tube de soupape, referme celle-ci, dans laquelle l'élément de ressort est un ressort coudé, un ressort articulé (9) dont une branche repose sur toute la longueur contre la paroi intérieure de la chambre (6),
**caractérisé en ce que** les branches (10, 11) de l'élément de ressort sont sur toute la surface et que la branche flexible (11) présente des épaulements (15, 16) sur les deux côtés longitudinaux.

2. Soupape de cathéter selon la revendication 1, **caractérisé en ce que** respectivement deux épaulements (15, 16) distants l'un de l'autre sont prévus sur les deux côtés longitudinaux de la branche (11).

3. Soupape de cathéter selon la revendication 1 ou 2, **caractérisé en ce que** l'organe d'actionnement (13) présente des branches latérales (17) avec des faces frontales (18) allongées, qui sont dirigées dans l'espace intermédiaire entre les épaulements (15, 16) de la branche (11) de l'élément de ressort.

4. Soupape de cathéter selon la revendication 1 ou 2, **caractérisé en ce qu'**un bouchon (3) est prévu, qui peut être relié au logement (1) par adhérence de forces et/ou complémentarité de formes.

5. Soupape de cathéter selon la revendication 4, **caractérisé en ce que** la tubulure d'insertion de tube (2) est dotée d'au moins une bague d'étanchéité (19).

6. Soupape de cathéter selon la revendication 1 ou 2, **caractérisé en ce qu'**un moyen (22) est appliqué latéralement sur le logement (1) et agit sur le tube de soupape (4) dans le sens longitudinal.

7. Soupape de cathéter selon au moins l'une des revendications 1 à 3, **caractérisé en ce que** l'élément de ressort est un ressort articulé (9).
